# EUROPEAN PATENT APPLICATION

(11) **EP 3 184 648 A1**
(43) Date of publication of application: **28.06.2017**
(21) Application number: 16185408.8
(22) Date of filing: 23.08.2016
(51) Int. Cl.: C12Q 1/68

(54) **ANALYTICAL PROCESS FOR GENOTOXICITY ASSESSMENT**

(30) Priority: 23.12.2015 EP 15202623
(71) Applicant: Medizinische Hochschule Hannover, 30625 Hannover (DE)
(72) Inventor: Rothe, Michael, 30173 Hannover (DE); Schambach, Axel, 30625 Hannover (DE); Baum, Christopher, 30625 Hannover (DE); Modlich, Ute, 63225 Langen (DE); Schwarzer, Adrian, 30161 Hannover (DE)
(74) Representative: Taruttis, Stefan Georg

(57) **Abstract**

The present invention relates to an analytical process for the assessment of the genotoxic potential, also termed mutagenic or transforming activity, of an agent in respect to hematopoietic cells. The agent can be selected from natural and synthetic chemical compounds, and preferably from heterologous expression cassettes, e.g. contained in non-natural viral particles or non-natural viral nucleic acid constructs, e.g. nucleic acid constructs containing a viral element.

## Description

The present invention relates to an analytical process for the assessment of the genotoxic potential, also termed mutagenic or transforming activity, of an agent in respect to hematopoietic cells. The agent can be selected from natural and synthetic chemical compounds, and preferably from heterologous expression cassettes, e.g. contained in non-natural viral particles or non-natural viral nucleic acid constructs, e.g. nucleic acid constructs containing a viral element. The heterologous, e.g. viral element can e.g. be a promoter or enhancer region for transgene expression, a viral primer binding site, a viral 5' region containing a U3 region, and/or a viral packaging signal.

The analytical process is suitable for assessing the genotoxic potential of viral particles for mammalian cells, e.g. for viral particles containing a nucleic acid construct intended for gene therapy. The hematopoietic cells are primary stem cells and/or stem precursor cells of the hematopoietic system, e.g. of human, murine or rat origin. The process is an in vitro process, using the primary stem cells and/or stem precursor cells of the hematopoietic system.

### State of the art

Modlich et al., Blood 108 (8), 2545-2553 (2006) describe that gene transfer to primary murine lineage negative bone marrow cells by adding viral nucleic acid constructs containing a SIN (self-inactivating 3'LTR upon integration into the host genome) nucleic acid construct resulted in lower frequencies of replated cells than an LTR-driven nucleic acid construct after 28 days under cell culture conditions. Higher replating frequencies in the cell culture were detected as cell growth in a limiting dilution series and were found to be indicative of a higher transforming potential of the viral particle. A disadvantage of this method is that the increased cell growth which is determined as an indicator for transformation strongly depends on the myeloid cultivation conditions. The replating phenotype is triggered or accompanied by activation of a very limited number of proto-oncogenes (mainly Mecom). Insertional upregulation of genes of the lymphoid differentiation lineage cannot be detected. A further disadvantage is that the cultivation of cells takes a long time and that the microscopic analysis of cell growth yields no information about the underlying transforming event.

### Object of the invention

It is an object of the invention to provide an alternative process for analysis of the genotoxic potential of an agent in mammalian hematopoietic cells. Preferably, the alternative process can be performed in a shorter time and/or yields a more detailed picture of the genotoxic potential.

### Description of the invention

The invention achieves the object by the features of the claims, especially by providing a process for analysis of the genotoxic activity of an agent for hematopoietic cells, the process comprising the steps of adding the agent to hematopoietic stem and progenitor cells in a cell culture under cell culture conditions, cultivating these cells for a pre-determined duration to produce cultivated cells, isolating total RNA, preferably total mRNA, from the cultivated cells and determining the concentration of the RNAs encoding at least one, preferably all of the RNA sequences of Table 1, preferably using the indicated probe sequences. Generally preferred, the RNAs are mRNAs.

**Table 1: RNA and preferred specific probe sequence**

| RNA name | probe sequence | Probe sequence SEQ ID NO: |
|---|---|---|
| AFAP1L1 | TAGTTATCTTTGCCTTCTGATGCAATGAACGCAC GTAAGCCATTCCATCCTTTCCTTGGC | 1 |
| ALS2CL | CCCTACTTTTCTGGGTGATGGCCACAATTACAGT CTACTTATTTATTCAGGATTTTTAAT | 2 |
| ARX | GTGTAATTGTTATCACTTTTCCTTGCTATCTAGTG GAGAAGTGTCACGCTCAAAATAAAA | 3 |
| BEX6 | ATGCTTACTGATGCCATACCGACAATAGCCAAGA TATGCAGTCAGCTTGGAGGTTCAACA | 4 |
| CCDC102A | TTTCACCTATCACACCCCTGTGAAGGGGGAGCCT GTTGGATATGTATATTGTATATATGC | 5 |
| DCTD | GCACTGTTAAATTTATCCGAGGCATAAGTAGTTC CTAGAGGAGACAGTCTATCACTCCCC | 6 |
| EHD3 | GCTTATAACTGCAGTGTGTTTGTCATTAGGATTC ATGTTAATACAACATATTTACCCTCG | 7 |
| GM805 | ATTTTGATGATTCAAGAAAGCAGAAGGCCCAAC CTTCAGTAAAACCACAGGTCTCCGGGA | 8 |
| GUCY1A3 | GGAACTTTATCTAATTCAAGTCAAAAGAAGTATA GAAGCTCCATGTACGGTTCCTATAAC | 9 |
| MEX3A | GCAAAGTAAAGGTGGGAAATAAAACAGACCCAT GAATTAATCAAGTCAAAGTGATGTTGC | 10 |
| MYCT1 | GTCCTTTTGCTTCAAAGACAGGAGTTCACTGGGG GATTCAGAAAGCTTGCCGTTTATGTT | 11 |
| NAIP1 | AATCTATGAGTTTTATGAACTCTCTAGGGGGCTT AACAACCCCTCACTGGGCAGTGTCAT | 12 |
| NOLC1 | TTCGAAGTAAGCTTTTCTGACAGACATTTTGCAA CAACTTGACTGTTGTATATTGACAAG | 13 |
| PA2G4 | GAATGGATCCTCATTTGTAAAAATTTTTAGCCAT TTTTGTTCTGAACAGTCCCTCAATCC | 14 |
| PDGFRB | CCACATTTAGACACCGGAAGTGCTATTTATATG CTGTTAAGTTTTCCTATCTGTACTTT | 15 |
| PHLDA2 | ACCATTCCGTGTTAATATTTTTTATACCATATTTT CATTCCAAATAAACAATGTCACTTT | 16 |
| SLA2 | CAAAGATACACCTCCACCTGTGACTGTGCCAACA TCATCACTAAATTGGAAAAAGCTGGA | 17 |
| SLC12A2 | CTGCTTTGTACAGAAGTTACTGCAATAAAGGAAG TGGAGTCATCACAAGTTTAATACTTA | 18 |
| SOX14 | TGGACCCTGCGCAATTTAGTTCCAGTGCCATCCA GAAGATGGGTGAAGTGCCCCACACGT | 19 |
| SOX4 | AATCCTGTCCGTCACTGCCTGTCAGGTTGTTCCT ATATACCTTCTGTAAATAACTTTTTT | 20 |
| SPNS2 | GCTGCTGATTGTGAATCTCAAAGTCTTAAGAGAG AAGCCAAATATATATTCCTCTTGTAA | 21 |
| TBXA2R | TGAGTGCTTGGTGGACTAAGGACGGAGCTACGA CATGGGGCTGACTCCTGTGAACTGCAA | 22 |
| TC1649157 | TAGAGAGAGTGAAACCTTGTTCTTGCTGAGTCTT CGAGAAAGAGGACCAAGTCCCAAAGT | 23 |
| TIE1 | GTGAACATGTCGCTGTTTGAGAACTTCACCTATG CGGGCATCGATGCCACAGCTGAGGAG | 24 |
| TMEM176A | GGTATTTTCTCGGAGATGATGTCTGTCAAAGAGA CTCTTCATATGGATGGTCCACCATGC | 25 |
| TMEM176B | CCTGAAGAAGTTTCTCTCCTGGCCTCAGGCCAGA ATCCACTATGGGCAGCTGTCTCTAGG | 26 |
| TOX | TCCAAGTCCTGTTATGGATAAAGCTGCCAAATGC TCACTGGCATCTTAGCTGCAGAAACA | 27 |
| ZFPM1 | AGTGAGCCTGTGGGGCCGCACCACCAGGACCCT TGACACTTAATAAAGACATTCGGTGTG | 28 |

and preferably relating the concentrations of the RNAs from cultivated cells to which the agent was added to the concentrations of RNAs determined for cells cultivated under the same cell culture conditions for the same duration in the absence of the agent.

Preferably, the concentration of at least one or all of the RNAs of Table 2 is additionally determined:

**Table 2: additional RNA and preferred specific probe sequence**

| RNA name | probe sequence | Probe sequence SEQ ID NO: |
|---|---|---|
| AJUBA | GGAAGCTGGAGAGAAGAAAAATAGGTTGACTTG GTTCTAGCCGCTTGGCTGAAACTTGAA | 29 |
| BHLHA15 | TCCCTGAGTACTAGTAAAATTGCCATCTGTACCT GAAAATGAAGACTTCACAGCCATTGT | 30 |
| CP | TCTGATGTCTTTGACCTTTTCCCTGGAACATACCA AACCCTAGAAATGTTTCCCCAAACA | 31 |
| ENSMUST00 000103558 | TAACGACACATACATGAAGTTTAGCTGGCTTACA GTGCCTGAAAGGGCAATGGGGAAAGA | 32 |
| GLRP1 | GGGGAAAAAAGCATCATTTCCATTGTTCTTTGCT CTGTGCCTTGAATCTTTTAATCAATT | 33 |
| GRTP1 | AAGCAGATCACCAAAGGGGACTTTGTGACAGAG TGTCACGCATTCATGCAGAAAATCTTT | 34 |
| LCK | AATCTTATGTCTCTGTGTGTTCTGTCCTGGTGCCT AGCACACACCAGGAGCTCAATAAAA | 35 |
| NAP002592-003 | TTCCCCTTCGAAAATAGGTGTTATAGTCAAGATA ATGTACTTGGGTCATAAGACAGTGTG | 36 |
| PIGA | CCAGTGTGCTAAAACATGCTCATGTCCAGTAATA AAGATGAGAACGCTGTTTTAATAGTC | 37 |
| PRMT7 | TGGAGACATCACCATGGAGTTTAGGCTTGCAGAC ACCTTGAGCTGATCTCTTATTGAGAA | 38 |
| SLC35F2 | CAGCCACCAGAGTGCCGCCTTCAGAAAACTTTAT GTAAATTGTTTTGTATAAGAATAAT | 39 |
| TGM2 | AAAGCGTGGAAGCCTGTGTGGGGGTCATCATCC CAAGTTAGCCCACTCCTACCTCTCTGT | 40 |
| TIMP3 | ATAGTGACTTTTGGGGATAGATCTGTCTGGGAGA TAATGTGAGTCCAGGAGGCATTACTC | 41 |
| ZFP184 | GGGATGGAGAGCCCATTGGGCTGTTCTGATCCAA GTATGTTATTAAAACAACATTGTGGC | 42 |
| AKAP13 | TTAGTCGCACCTTCAGCTACATCAGGAATAAAAT GTCCAGCAGCAAGAAGAGCAAAGTAA | 43 |
| ASB2 | CCTGGACACACTGCCGCTTCCCGGCAGGCTAATC AGATACTTGAAATATGAGAATACACA | 44 |
| ATP9A | TTCAACTTCTACTTCCTGCTTCTCGCCTGCTCGCA GTTCGTCCCAGAGATGAGGCTTGGC | 45 |
| CCR7 | CAGCCACTGATACCTTTCCTCATGTTCTGCTTTTG ATTCATATATCTTTTATGAAGAAAC | 46 |
| CPLX2 | GGAGGAGAAGGAGAAGGAGAAGGAGAAGGAGA AGGAGAAGGAGAAGGAGAAGGAGAAGGA | 47 |
| DUSP2 | GTGGGCATCTCGCTGTAATTGGTGCTGAAAAGTT ATTTGTGTTCAACTGACATTTAACGC | 48 |
| ELOVL6 | CCTAGGCCCTAAACCCATATTCAGAGGGAAAATT CACTATCAAGCCTCACAGCGAAATCA | 49 |
| EPOR | GAACGGGATTGGTGAAGCCATACTTAAAGTCAG AGCTGACCTTGGCCCTCTGAGCAGGAA | 50 |
| FGF3 | GCTGTATGCTTCGGATCACTACAACGCAGAGTGT GAGTTTGTGGAACGGATCCATGAGCT | 51 |
| IGF1R | GCTAAAGCTAAGGCCACTTGAGTCTATTACTCTG CTTTTTTCTAGTAGTTAAAGCACCAC | 52 |
| ITK | ACTGCAGGTATGTCTTACCCTCTGTGGGGGCCAC AAGTCAATCATTGCTTATGGAAAAAA | 53 |
| JAKMIP1 | CTGTACCAGAGCAATCTATTTATTGCCTACCGCT TGTTTTGCACTTAATAAAATAAGTTG | 54 |
| KLRB1F | GGTTTGTATTGATTGACACTCGCCAACTAGTTGA CTAATAATGGACTAATGCTGTGTTCG | 55 |
| LMO2 | CTTCATTTTGAGGTGAGGCGCCCCACAGATTGTT TCTATACAAATGTAAATCTTAAAAAA | 56 |
| MEF2C | AGAGGAATAGTATGTCTCCTGGTGTAACACATAG ACCTCCAAGTGCAGGTAACACAGGTA | 57 |
| MID1 | AGGTTGGATGTCACCACCACAAGGGAAAACAAA CACATGGGTTTTCCCCAACACGATTA | 58 |
| MND1 | GGCAAGAGACGGAAGAACGAGCCATGCTTGCAA AAGAACTTTCTTCATTTCGAGACCAAA | 59 |
| MYB | TGACAGTGTATCTACTGCCTTGTAGCAAAATAAA GCTATCCTCTTATTTACATACTTCC | 60 |
| MYC | AACAGTTGAAACACAAACTCGAACAGCTTCGAA ACTCTGGTGCATAAACTGACCTAACTC | 61 |
| PIM2 | CACGCTACAGGGATAGATGGACATCTGTTGACCT GGTTATACAGGTTGTTAAAAATCCAG | 62 |
| PRDM16 | CTGGATACCAAATGTAATCTTTCCGATGCTACAA TGAATTTATACACGAGATTGATATGC | 63 |
| RAMP1 | AGACGCTATGGTGTGACTGGGGAAAGACCATAC AGAGCTATGGGGAGCTCACTTACTGCA | 64 |
| RASGRP2 | TCTGTTAGACCAAGAAGGGAACCGCAGGCACAG CAGCCTCATCGACATCGAGAGTGTGTG | 65 |
| SEMA7A | TCCCTTATCTCTTCTCAATGCACTTTAATAATGTA ACATATTACTAATAAACAAGCTATT | 66 |
| SH3KBP1 | GCCAATTAAACTAAGACCAAGGTCGATTGAAGT GGAAAATGACTTCCTGCCCGTCGAAAA | 67 |
| SLC43A1 | TGCACACATATATGTTACGCACATGCATATTGCA TCCTTTCCTGCAGGAGAAGGACTGAG | 68 |
| SMAD3 | TATAAACGCCCTTCTAATAAACTTTTCACCGTAA AGCTCCTGAGACAGGAGCACAGTCTG | 69 |
| ST3GAL6 | AATGGTGATCAACTTGACTTAAAATTGACCCTAT GGATCCAAAAGATGATGATGCTAAAC | 70 |
| TNFSF13B | CGTTGAATCTGATCCAAACCAGGAAATATAACA GACAGCCACAACCGAAGTGTGCCATGT | 71 |
| VARS | TGCTCTTGAACTAGCTCTGAGCATCACTCGAGCT GTGCGCTCCCTGCGTGCTGACTACAA | 72 |

The step of relating the concentrations of the mRNAs determined for the cells to which the agent was added with subsequent cultivation to the concentrations of the mRNAs determined for the same type of cells which were cultivated without addition of the agent under the same cell culture conditions results in the determination of differences in the expression levels of these RNAs, which differences in expression levels are characteristic for the genotoxic effect of the agent. As the expression levels of mRNAs have been found to depend on the culture conditions, the cells are cultivated under the same culture conditions and the same duration for each comparison, preferably performed in parallel each time. The cells cultivated under the same cell culture conditions for the same duration in the absence of the agent are also referred to as a negative control or mock.

Preferably, an agent that is known to have a significant genotoxic potential is used under the same culture conditions to provide a positive control, preferably performed each time in parallel to the cells to which the agent was added, and the process comprises the step of comparing the concentrations of the mRNAs determined for the positive control to the concentrations of mRNAs determined for the cells to which the agent to be analysed was added. Generally, the process preferably comprises the comparison of expression levels of the RNAs determined for the cells to which the agent was added to the expression levels of the cellular RNAs determined for the negative control and/or of the positive control, wherein all cells were cultivated under the same cell culture conditions for the same pre-determined duration.

It has been found that the determination of the concentration of these mRNAs allows the differentiation between immortalized cells and non-immortalized cells. Therein, immortalized cells are considered to represent precancerous cells and/or tumour cells, indicating a high genotoxic activity of the agent analysed in the process. Specifically, it has been found that expression levels of the RNAs that are determined for the cells to which the agent was added, which expression levels have differences in comparison to the expression levels of the mRNAs determined for the negative control and/or no or less important differences in comparison to a positive control, are characteristic for the genotoxic activity of the agent. Using a viral particle known to have genotoxic activity as an exemplary agent and murine hematopoietic stem cells and their progenitor cells, significant differences in expression levels of the RNAs were found in comparison to the expression levels determined for the RNAs in a parallel negative control.

The step of relating the concentrations of the mRNAs determined for the hematopoietic cells to which the agent was added to the concentrations of mRNAs determined for the hematopoietic cells cultivated under the same cell culture conditions for the same duration in the absence of the agent preferably is by calculating the ratio of the specific mRNA concentrations. Generally, the concentration ratio can be replaced by the ratio of measurement values measured for the mRNA concentrations, e.g. by the ratio of fluorescence measurement values. Preferably, only the mRNAs are considered as having an expression level which significantly differs from the expression determined for the negative control. For example, a significant difference can be defined for a difference of at least a log₂ of the concentration ratio bigger than +1 or smaller than -1. This exemplary cut-off for expression levels which compared to the negative control differ at least by such a factor can be used in order to limit the RNAs to those which have an important difference in expression level.

Preferably, for agents containing a nucleic acid, the copy number of this nucleic acid in relation to the genome of the cells is determined. In this manner, the influence of the copy number can be taken into account in the determination of the genotoxic risk of an agent. For agents not containing a nucleic acid, the concentration of the agent should be measured in order to take into account the concentration effect, for example by dividing the concentration of RNAs determined in the cells with agent by the concentration of the added agent.

The culture conditions comprise the composition of the cultivation medium, cultivation temperature, the composition of the gas atmosphere, and the movement of the cultivation medium, e.g. by shaking, stirring, or static incubation.

A preferred medium for the hematopoietic stem cells and their progenitor cells used in the process contains growth-promoting cytokines, especially the following cytokines: stem cell factor (SCF), preferably in addition interleukin-3 (IL-3), Flt3-ligand, interleukin-11 (IL-11), and more preferably additionally interleukin-7 (IL-7), and further optionally additionally interleukin-6 (IL-6), interleukin-15 (IL-15), thrombopoietin (TPO) and further optionally additionally at least one notch ligand (DL1 or DL4).

An advantage of the process of the invention is that it is not restricted to measure presence of cells that proliferate during a long period of cultivation as e.g. in the process of Modlich (2006), as it has been found that the RNA isolated in the process according to the invention does not necessarily contain, and preferably not contain relevant levels of the mRNA Lmo2, whereas in cells treated in the process of Modlich (2006) all cells that finally proliferated, when analysed by the process of the invention were found to express Lmo2. Therefore, the process of the invention has the advantage of identifying Lmo2, which is an indicator for precedence of adverse events in gene therapy, e.g. an indicator associated with the development of leukemia by gene therapy. As a further advantage, the process is not dependent on the expression of Lmo2 by the cells to be analysed.

Following addition of the agent to the cells in cell culture and prior to cultivating the cells, the process can comprise the step of incubating the cells in the presence of the agent and removing the agent, e.g. by exchanging the culture medium of the cells in the cell culture for fresh medium without the agent, e.g. following a prior incubation period of 1 to 3 d.

Preferably, the agent is a non-natural nucleic acid construct, optionally packaged as a viral particle. The non-natural nucleic acid construct can e.g. contain a non-natural or a natural nucleic acid sequence having the ability to integrate into genomic DNA, e.g. at least a sequence having homology to the cell, a viral primer binding site, a viral 5' region containing a U3 region, and/or a viral packaging signal promoter sequence.

When the agent comprises a nucleic acid construct, e.g. the agent is a viral particle, the process preferably comprises the step of performing a quantitative analysis of the nucleic acid construct in total DNA obtained during the pre-determined duration from an aliquot of the cultivated cells for determination of the average copy number of the agent in the mammalian cell. Preferably, the quantitative analysis of the nucleic acid construct of the agent in total DNA obtained from the cells is made by quantitative PCR (polymerase chain reaction) using a primer pair specific for the nucleic acid construct, and preferably also a primer pair specific for a genomic DNA section of the cells, in a separate or in the same PCR. The step of collecting DNA from the cells for the quantitative analysis of the nucleic acid construct can e.g. be made at 2 to 8 d, preferably 4 to 8 d, more preferably 4 d following the addition of the agent to mammalian cells in a cell culture, in order to avoid detection of viral nucleic acids from episomes or from transiently infected cells.

The pre-determined duration of cultivating the cells following the step of adding the agent to the cells in culture can e.g. amount to 10 to 15 days, and this duration can optionally be divided into a first and a second partial duration, wherein the first partial duration of cultivating the cells is followed by a step of diluting the cells and then by cultivating the cells for the second partial duration, e.g. by re-plating the cells at lower cell number and cultivating for a second partial duration. In this embodiment, the step of diluting the cells for a second partial duration of incubation can increase the selection pressure on the cells and therefore result in more pronounced differences in the translation of mRNA species. Preferably, the first partial duration can be 2 to 10 d, preferably 6 to 8 d, and the second partial duration that follows the dilution of the cells for further cultivation can be 5 to 12 d, e.g. 5 to 7 d.

Optionally, the pre-determined duration of cultivating the cells, optionally the first partial duration can be 2 to 6 d, preferably 3 to 4 d. Such duration can have the advantage of determining concentrations of mRNAs, which preferably are mRNAs, characteristic for general toxicity of agents, whereas longer durations, especially durations of 8 to 15 d, have been found to result in specific differences in expression levels of the mRNAs regarding genotoxicity. This shows a specific advantage of the process of the invention in that it generally results in the identification of genotoxic activity of an agent at higher sensitivity and identification but also the identification of a general toxicity of an agent that prevents or reduces proliferation of the cells.

A further advantage of the process of the invention is that it does not require the replating step needed for microscopic analysis of transformed or immortalized cells and hence can use shorter pre-determined durations of cultivating cells as e.g. used in the process of Modlich et al. (2006), which uses an expansion plus limiting dilution of the murine hematopoietic stem and progenitor cells in culture for 4 weeks following transduction of cells.

The primary hematopoietic stem cells and/or stem progenitor cells obtained freshly or used after a freeze-thaw-cycle, which are used in the process of the invention, e.g. of murine origin, can be cultivated under cell culture conditions prior to adding the agent, adding nothing or an inactive compound for the negative control, and adding a known genotoxic agent as a positive control, respectively, to separate cells in culture at the same prior incubation period. This cultivation period prior to adding the agent can e.g. last for 1 to 3 d, e.g. 2 d for freshly obtained primary cells.

The step of determining the concentration of the mRNAs can be by reverse transcription followed by quantitative PCR, preferably by hybridisation of the mRNA, preferably the total RNA isolated from the cultivated cells, to nucleic acid probes which are immobilized on a substrate, e.g. arranged on a substrate as an array of probes. Arrays of nucleic acid probes immobilized on a substrate are generally known as microarrays which are suitable for quantification of hybridizing mRNAs. Optionally, concentration of mRNAs can be quantified by next generation sequencing of the reverse transcribed RNAs.

In an optional embodiment, the process contains the evaluation of the mRNAs which are considered as deregulated. Therein, the mRNAs are considered deregulated for which subsequent to normalization, preferably Quantil normalization (e.g. performed using the R-package limma - linear models for microarrays), for the concentration ratio with the negative control a log₂ of bigger than +1 or smaller than -1 is found.

Preferably additionally, a normalized enrichment score (NES) is calculated for each of the mRNAs considered deregulated. The NES is calculated as described by Subramanian et al., Proc Natl Acad Sci USA (2005) Oct 25; 102(43):15545-50. In short, the NES is calculated by the steps of generating a numerical matrix of the protein encoding genes, creating a running-sum statistic with a maximum deviation from zero (Enrichment Score), determination of a nominal P value (Estimation of Significance Level) and adjusting the Enrichment Score for Multiple Hypothesis Testing (Normalized Enrichment Score). Therein, an NES above 2.0 indicates a high probability that the agent has transformed the cells, an NES below 1.2 indicates a low probability that the agent has genotoxic potential, and an intermediate NES, i.e. between 1.2 and 2.0 indicates that the agent has potential genotoxic activity.

Preferably, in a subsequent step databanks are searched for the mRNAs considered deregulated, the databanks containing RNAs, which preferably are mRNAs, which have been found associated with diseases, e.g. relevant in diseases, e.g. in tumour patients, e.g. in AML, ALL or MDS patients, or in mouse models of such a disease. A hit in each databank for an RNA considered deregulated adds weight to this RNA as the overlap percentage for a hit in the databank. The result of the addition of the weight, e.g. the percentage of overlap to the at least one databank, e.g. to the log₂ ratio for each mRNA, yields a numerical value reflecting the genotoxic potential.

It was found that this calculation of a value reflecting the genotoxic potential for viral particles which are currently considered as having a low genotoxic potential yields values close to zero, and that this calculation for known mutagenic viral particles yields values which are significantly bigger.

The databanks contain genes which when deregulated can have a genotoxic effect at least of viral particles. The databanks, e.g. at least one, are selected from the following ones:
1. Retroviral Tagged Cancer Gene Database (RTCGD, available from http://variation.osu.edu/rtcgd/) containing insertions detected in the vicinity of genes caused by retrovirus (RCGD-retro) or by transposons (RTCGD-transposon) in at least one murine tumour.
2. Bushman Cancer Gene List (available from www.bushmanlab.org/links/genelists) containing gene names of several cancer data bases, e.g. Atlas, CANgenes, CIS (RTCGD), human lymphoma-associated genes, a list of retrovirally induced tumours, the Sanger Cancer Development List, the Vogelstein List of chromosomal aberrations having importance in cancer development.

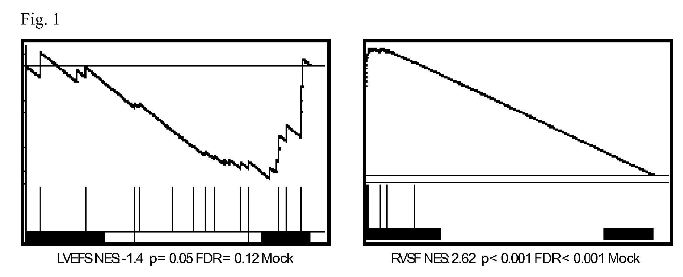

The invention is now described by way of examples with reference to the figure, which shows in Fig. 1 a graphic representation of exemplary NES values determined for an exemplary agent by a preferred embodiment of the process.

### Example 1: Analysis of genotoxic potential by determination of mRNA concentrations in cultivated cells

Murine primary hematopoietic stem precursor cells that were isolated, cryopreserved, thawed, cultivated for 3 d in serum-free medium, preferably in StemSpan medium containing SCF, Flt3-L, IL-3, IL-11, IL-7 and expanded in IMDM medium (same cytokines) for 2 d at 37°C, 5% CO₂ atmosphere in 24-well cell culture dishes. At day 0, the agent, a positive control and medium as a negative control were added to separate dishes of the cultivated cells. The exemplary agent suspected of having a genotoxic activity was a viral particle containing a nucleic acid construct coding for the IL-2 receptor common gamma chain under an elongation factor 1 alpha short promoter, designated LVEFS. The positive control was a viral particle containing a nucleic acid construct with intact LTR regions (promoter/enhancer regions) from the spleen focus forming virus, designated RVSF.

The cells with added agent, positive control and negative control, respectively, were incubated under cell culture conditions for 4 days, then an aliquot of each cell culture was removed for extraction of genomic DNA, from which the copy number of the viral nucleic acid sequence was determined. In detail, the primers of Table 3 were used to detect the viral elements indicated.

**Table 3: primers for detecting viral elements of a viral particle**

| viral element | function of primer or probe | primer or probe | SEQ ID NO: |
|---|---|---|---|
| WPRE | forward | GAGGAGTTGTGGCCCGTTGT | 73 |
| WPRE | reverse | TGACAGGTGGTGGCAATGCC | 74 |
| WPRE | Taqman Probe | CTGTGTTTGCTGACGCAAC | 75 |
| PTBP2 | forward | TCTCCATTCCCTATGTTCATGC | 76 |
| PTBP2 | reverse | GTTCCCGCAGAATGGTGAGGTG | 77 |
| PTBP2 | Taqman Probe | ATGTTCCTCGGACCAACTTG | 78 |

The cultivated cells were expanded in IMDM medium containing cytokines SCF (100 ng/ml), IL-3 (20 ng/ml), Flt-3L (100 ng/ml), IL-11 (100 ng/ml), and IL-7 (100 ng/ml) for further 4 days, then collected and re-plated at a 1:10 dilution in fresh medium of this composition and cultivated for another 7 d. Alternatively, the medium in addition contained OP9-DL1 or DL4 at a concentration of 5 µg/ml. Then total RNA was collected from the cells using RNAzol and the extraction kit from Zymo Research.

The RNA from each culture was analysed for the concentration of the following RNAs of Table 1: AFAP1L1, ALS2CL, ARX, BEX6, CCDC102A, DCTD, EHD3, GM805, GUCY1A3, MEX3A, MYCT1, NAIP1, NOLC1, PA2G4, PDGFRB, PHLDA2, SLA2, SLC12A2, SOX14, SOX4, SPNS2, TBXA2R, TC1649157, TIE1, TMEM176A, TMEM176B, TOX, ZFPM1. Additionally, the following RNAs of Table 2 were analysed: AJUBA, BHLHA15, CP, ENSMUST00000103558, GLRP1, GRTP1, LCK, NAP002592-003, PIGA, PRMT7, SLC35F2, TGM2, TIMP3, ZFP184, AKAP13, ASB2, ATP9A, CCR7, CPLX2, DUSP2, ELOVL6, EPOR, FGF3, IGF1R, ITK, JAKMIP1, KLRB1F, LMO2, MEF2C, MIDI, MND1, MYB, MYC, PIM2, PRDM16, RAMP1, RASGRP2, SEMA7A, SH3KBP1, SLC43A1, SMAD3, ST3GAL6, TNFSF13B, VARS. For analysis of the RNAs, hybridisation to immobilized specific oligonucleotides contained on a chip, followed by optical detection using laser irradiation of the fluorescent dye Cyanine 3 were used.

The ratio of the mRNA concentrations were determined as the quotient of the fluorescence signal intensity determined for the cells to which the agent was added to the signal intensity determined in the negative control for each mRNA species.

The differentiation between immortalized cells and non-immortalized cells was verified by performing the conventional limiting dilution protocol of the Modlich (2006) assay in parallel. Differences between immortalized and non-immortalized cells can be assessed by the measurement of the mRNA species of Table 1. For determination of the numerical value discriminating the genotoxic potential, a normalized enrichment score (NES) was calculated for the significantly expressed mRNAs using the GSEA method. The exemplary agent LVEFS had a NES of -1.4 which was significantly different from the positive control RVSF with an NES of 2.62.

For determination of the genotoxic potential according to the database RTCGD, the significantly increased concentrations of RNA species of Table 1 and of the additional RNAs of Table 2 that were determined in the cells to which the exemplary agent LVEFS or RVSF was added, were analysed for overlaps with the database. The sample LVEFS had a mean overlap of 0 out of 44 and the RVSF 17 out of 44. This corresponds to a p-value < 0.001 and is regarded highly significant.
Fig. 1 shows the normalized enrichment scores of sample LVEFS (-1.4) and the positive control RVSF (2.62).

## Claims

1. Process for analysis of the genotoxic activity of an agent for primary hematopoietic cells, the process comprising the steps of adding the agent to the primary hematopoietic cells in culture under cell culture conditions, cultivating the cells for a pre-determined duration to produce cultivated cells, **characterized by** the step of isolating total RNA from the cultivated cells, determining the concentration of at least one, preferably all, of the RNAs selected from the group comprising
| | |
|---|---|
| AFAP1L1, | PDGFRB, |
| ALS2CL, | PHLDA2, |
| ARX, | SLA2, |
| BEX6, | SLC12A2, |
| CCDC102A, | SOX14, |
| DCTD, | SOX4, |
| EHD3, | SPNS2, |
| GM805, | TBXA2R, |
| GUCY1A3, | TC1649157, |
| MEX3A, | TIE1, |
| MYCT1, | TMEM176A, |
| NAIP1, | TMEM176B, |
| NOLC1, | TOX, and |
| PA2G4, | ZFPM1, |
and relating the concentrations of the mRNAs to the concentrations of mRNAs determined for cells cultivated under the same cell culture conditions for the same duration in the absence of the agent.

2. Process according to claim 1, **characterized in that** the RNA is detected by hybridization to a probe having a nucleic acid sequence
| Specific for RNA named | probe sequence | Probe sequence SEQ ID NO: |
|---|---|---|
| AFAP1L1 | TAGTTATCTTTGCCTTCTGATGCAATGAACGCA CGTAAGCCATTCCATCCTTTCCTTGGC | 1 |
| ALS2CL | CCCTACTTTTCTGGGTGATGGCCACAATTACAG TCTACTTATTTATTCAGGATTTTTAAT | 2 |
| ARX | GTGTAATTGTTATCACTTTTCCTTGCTATCTAGT GGAGAAGTGTCACGCTCAAAATAAAA | 3 |
| BEX6 | ATGCTTACTGATGCCATACCGACAATAGCCAAG ATATGCAGTCAGCTTGGAGGTTCAACA | 4 |
| CCDC102A | TTTCACCTATCACACCCCTGTGAAGGGGGAGCC TGTTGGATATGTATATTGTATATATGC | 5 |
| DCTD | GCACTGTTAAATTTATCCGAGGCATAAGTAGTT CCTAGAGGAGACAGTCTATCACTCCCC | 6 |
| EHD3 | GCTTATAACTGCAGTGTGTTTGTCATTAGGATTC ATGTTAATACAACATATTTACCCTCG | 7 |
| GM805 | ATTTTGATGATTCAAGAAAGCAGAAGGCCCAA CCTTCAGTAAAACCACAGGTCTCCGGGA | 8 |
| GUCY1A3 | GGAACTTTATCTAATTCAAGTCAAAAGAAGTAT AGAAGCTCCATGTACGGTTCCTATAAC | 9 |
| MEX3A | GCAAAGTAAAGGTGGGAAATAAAACAGACCCA TGAATTAATCAAGTCAAAGTGATGTTGC | 10 |
| MYCT1 | GTCCTTTTGCTTCAAAGACAGGAGTTCACTGGG GGATTCAGAAAGCTTGCCGTTTATGTT | 11 |
| NAIP1 | AATCTATGAGTTTATGAACTCTCTAGGGGGCT TAACAACCCCTCACTGGGCAGTGTCAT | 12 |
| NOLC1 | TTCGAAGTAAGCTTTTCTGACAGACATTTTGCA ACAACTTGACTGTTGTATATTGACAAG | 13 |
| PA2G4 | GAATGGATCCTCATTTGTAAAAATTTTTAGCCA TTTTTGTTCTGAACAGTCCCTCAATCC | 14 |
| PDGFRB | CCACATTTAGACACCGGAAGTGCTATTTATAT GCTGTTAAGTTTTCCTATCTGTACTTT | 15 |
| PHLDA2 | ACCATTCCGTGTTAATATTTTTTATACCATATTT TCATTCCAAATAAACAATGTCACTTT | 16 |
| SLA2 | CAAAGATACACCTCCACCTGTGACTGTGCCAAC ATCATCACTAAATTGGAAAAAGCTGGA | 17 |
| SLC12A2 | CTGCTTTGTACAGAAGTTACTGCAATAAAGGAA GTGGAGTCATCACAAGTTTAATACTTA | 18 |
| SOX14 | TGGACCCTGCGCAATTTAGTTCCAGTGCCATCC AGAAGATGGGTGAAGTGCCCCACACGT | 19 |
| SOX4 | AATCCTGTCCGTCACTGCCTGTCAGGTTGTTCCT ATATACCTTCTGTAAATAACTTTTTT | 20 |
| SPNS2 | GCTGCTGATTGTGAATCTCAAAGTCTTAAGAGA GAAGCCAAATATATATTCCTCTTGTAA | 21 |
| TBXA2R | TGAGTGCTTGGTGGACTAAGGACGGAGCTACG ACATGGGGCTGACTCCTGTGAACTGCAA | 22 |
| TC1649157 | TAGAGAGAGTGAAACCTTGTTCTTGCTGAGTCT TCGAGAAAGAGGACCAAGTCCCAAAGT | 23 |
| TIE1 | GTGAACATGTCGCTGTTTGAGAACTTCACCTAT GCGGGCATCGATGCCACAGCTGAGGAG | 24 |
| TMEM176 A | GGTATTTTCTCGGAGATGATGTCTGTCAAAGAG ACTCTTCATATGGATGGTCCACCATGC | 25 |
| TMEM176 B | CCTGAAGAAGTTTCTCTCCTGGCCTCAGGCCAG AATCCACTATGGGCAGCTGTCTCTAGG | 26 |
| TOX | TCCAAGTCCTGTTATGGATAAAGCTGCCAAATG CTCACTGGCATCTTAGCTGCAGAAACA | 27 |
| ZFPM1 | AGTGAGCCTGTGGGGCCGCACCACCAGGACCC TTGACACTTAATAAAGACATTCGGTGTG | 28 |

3. Process according to one of the preceding claims, **characterized in that** the concentrations of all the RNAs of claim 1 are analysed.

4. Process according to one of the preceding claims, **characterized by** additionally determining the concentration of at least one of the RNAs selected from the group comprising
| | |
|---|---|
| AJUBA, | CPLX2, |
| BHLHA15, | DUSP2, |
| CP, | ELOVL6, |
| ENSMUST00000103558, | EPOR, |
| GLRP1, | FGF3, |
| GRTP1, | IGF1R, |
| LCK, | ITK, |
| NAP002592-003, | JAKMIP1, |
| PIGA, | KLRB1F, |
| PRMT7, | LMO2, |
| SLC35F2, | MEF2C, |
| TGM2, | MIDI, |
| TIMP3, | MND1, |
| ZFP184, | MYB, |
| AKAP13, | MYC, |
| ASB2, | PIM2, |
| ATP9A, | PRDM16, and |
| CCR7, | RAMP1, |
and relating the concentrations of the mRNAs to the concentrations of mRNAs determined for cells cultivated under the same cell culture conditions for the same duration in the absence of the agent.

5. Process according to claim 4, **characterized in that** the RNA is detected by hybridization to a probe having a nucleic acid sequence
| Specific for RNA named | probe sequence | Probe sequence SEQ ID NO: |
|---|---|---|
| AJUBA | GGAAGCTGGAGAGAAGAAAAATAGGTTGACTTG GTTCTAGCCGCTTGGCTGAAACTTGAA | 29 |
| BHLHA15 | TCCCTGAGTACTAGTAAAATTGCCATCTGTACCT GAAAATGAAGACTTCACAGCCATTGT | 30 |
| CP | TCTGATGTCTTTGACCTTTTCCCTGGAACATACCA AACCCTAGAAATGTTTCCCCAAACA | 31 |
| ENSMUS T0000010 3558 | TAACGACACATACATGAAGTTTAGCTGGCTTACA GTGCCTGAAAGGGCAATGGGGAAAGA | 32 |
| GLRP1 | GGGGAAAAAAGCATCATTTCCATTGTTCTTTGCT CTGTGCCTTGAATCTTTTAATCAATT | 33 |
| GRTP1 | AAGCAGATCACCAAAGGGGACTTTGTGACAGAG TGTCACGCATTCATGCAGAAAATCTTT | 34 |
| LCK | AATCTTATGTCTCTGTGTGTTCTGTCCTGGTGCCT AGCACACACCAGGAGCTCAATAAAA | 35 |
| NAP0025 92-003 | TTCCCCTTCGAAAATAGGTGTTATAGTCAAGATA ATGTACTTGGGTCATAAGACAGTGTG | 36 |
| PIGA | CCAGTGTGCTAAAACATGCTCATGTCCAGTAATA AAGATGAGAACGCTGTTTTAATAGTC | 37 |
| PRMT7 | TGGAGACATCACCATGGAGTTTAGGCTTGCAGAC ACCTTGAGCTGATCTCTTATTGAGAA | 38 |
| SLC35F2 | CAGCCACCAGAGTGCCGCCTTCAGAAAACTTTAT GTAAATTGTTTTTGTATAAGAATAAT | 39 |
| TGM2 | AAAGCGTGGAAGCCTGTGTGGGGGTCATCATCC CAAGTTAGCCCACTCCTACCTCTCTGT | 40 |
| TIMP3 | ATAGTGACTTTTGGGGATAGATCTGTCTGGGAGA TAATGTGAGTCCAGGAGGCATTACTC | 41 |
| ZFP184 | GGGATGGAGAGCCCATTGGGCTGTTCTGATCCAA GTATGTTATTAAAACAACATTGTGGC | 42 |
| AKAP13 | TTAGTCGCACCTTCAGCTACATCAGGAATAAAAT GTCCAGCAGCAAGAAGAGCAAAGTAA | 43 |
| ASB2 | CCTGGACACACTGCCGCTTCCCGGCAGGCTAATC AGATACTTGAAATATGAGAATACACA | 44 |
| ATP9A | TTCAACTTCTACTTCCTGCTTCTCGCCTGCTCGCA GTTCGTCCCAGAGATGAGGCTTGGC | 45 |
| CCR7 | CAGCCACTGATACCTTTCCTCATGTTCTGCTTTTG ATTCATATATCTTTTATGAAGAAAC | 46 |
| CPLX2 | GGAGGAGAAGGAGAAGGAGAAGGAGAAGGAGA AGGAGAAGGAGAAGGAGAAGGAGAAGGA | 47 |
| DUSP2 | GTGGGCATCTCGCTGTAATTGGTGCTGAAAAGTT ATTTGTGTTCAACTGACATTTAACGC | 48 |
| ELOVL6 | CCTAGGCCCTAAACCCATATTCAGAGGGAAAATT CACTATCAAGCCTCACAGCGAAATCA | 49 |
| EPOR | GAACGGGATTGGTGAAGCCATACTTAAAGTCAG AGCTGACCTTGGCCCTCTGAGCAGGAA | 50 |
| FGF3 | GCTGTATGCTTCGGATCACTACAACGCAGAGTGT GAGTTTGTGGAACGGATCCATGAGCT | 51 |
| IGF1R | GCTAAAGCTAAGGCCACTTGAGTCTATTACTCTG CTTTTTTCTAGTAGTTAAAGCACCAC | 52 |
| ITK | ACTGCAGGTATGTCTTACCCTCTGTGGGGGCCAC AAGTCAATCATTGCTTATGGAAAAAA | 53 |
| JAKMIP1 | CTGTACCAGAGCAATCTATTTATTGCCTACCGCT TGTTTTGCACTTAATAAAATAAGTTG | 54 |
| KLRB1F | GGTTTGTATTGATTGACACTCGCCAACTAGTTGA CTAATAATGGACTAATGCTGTGTTCG | 55 |
| LMO2 | CTTCATTTTGAGGTGAGGCGCCCCACAGATTGTT TCTATACAAATGTAAATCTTAAAAAA | 56 |
| MEF2C | AGAGGAATAGTATGTCTCCTGGTGTAACACATAG ACCTCCAAGTGCAGGTAACACAGGTA | 57 |
| MID1 | AGGTTGGATGTCACCACCACAAGGGAAAACAAA CACATGGGTTTTTCCCCAACACGATTA | 58 |
| MND1 | GGCAAGAGACGGAAGAACGAGCCATGCTTGCAA AAGAACTTTCTTCATTTCGAGACCAAA | 59 |
| MYB | TGACAGTGTATCTACTGCCTTGTAGCAAAATAAA GCTATCCTCTTATTTACATACTTCC | 60 |
| MYC | AACAGTTGAAACACAAACTCGAACAGCTTCGAA ACTCTGGTGCATAAACTGACCTAACTC | 61 |
| PIM2 | CACGCTACAGGGATAGATGGACATCTGTTGACCT GGTTATACAGGTTGTTAAAAATCCAG | 62 |
| PRDM16 | CTGGATACCAAATGTAATCTTTCCGATGCTACAA TGAATTTATACACGAGATTGATATGC | 63 |
| RAMP1 | AGACGCTATGGTGTGACTGGGGAAAGACCATAC AGAGCTATGGGGAGCTCACTTACTGCA | 64 |

6. Process according to one of the preceding claims, **characterized in that** the agent is a virus or a viral particle containing a non-natural nucleic acid sequence, the process comprising the step of determining the copy number of the nucleic acid sequence of the viral particle in the genome of the cells.

7. Process according to one of the preceding claims, **characterized in that** the duration is 7 to 15 d, during which the cells are provided at least once with fresh culture medium.

8. Process according to one of the preceding claims, **characterized in that** under the culture conditions the medium contains cytokines consisting of the group of SCF, IL-3, Flt-3L, IL-11, IL-15, IL-6, IL-7, TPO, and Notch-ligands DL-1 and DL-4.

9. Process according to one of the preceding claims, **characterized in that** only those RNAs are considered deregulated for which following normalization the ratio of RNA concentration determined for the cells to which the agent was added to the RNA concentration of cells cultivated in the absence of the agent is significantly different.

10. Process according to one of the preceding claims, **characterized in that** a normalized enrichment score (NES) is calculated and that an NES above 2.0 indicates a high genotoxic activity and an NES below 1.2 indicates a low genotoxic activity of the agent.

11. Process according to one of the preceding claims, **characterized in that** for each RNA an overlap with a databank is determined and the total number of mRNAs overlapping with a database for the cells to which the agent was added and cells cultivated in the presence of the positive control is added to a numerical value reflecting the genotoxic potential, wherein the databank is at least one selected from the databanks comprised in the group of:
Retroviral Tagged Cancer Gene Database, and Bushman Cancer Database.

12. Process according to one of the preceding claims, **characterized in that** it includes separate treatment of identical primary hematopoietic cells by adding an agent of known genotoxic activity as a positive control, wherein the cells are cultivated under the same cell culture conditions and for the same duration, and determining the concentrations of the mRNAs for cells for the positive control.

13. Process according to one of the preceding claims, **characterized in that** the numerical value is calculated by a Fisher's exact test, where a p-value < 0.05 (agent vs. positive control) is regarded statistically significant, reflecting a lower genotoxic potential of the agent compared to the positive control.

14. Process according to one of the preceding claims, **characterized in that** the positive control is a viral particle containing a nucleic acid construct with intact LTR regions (promoter/enhancer regions) from the spleen focus forming virus.

15. Process according to one of the preceding claims, **characterized in that** the concentration of the RNAs is determined as the normalized enrichment score (NES) and a NES value above 2.0 is taken as a high probability for genotoxic potential, a NES value below 1.2 is taken as a low probability for genotoxic potential, and a NES value of 1.2 to 2.0 is taken as genotoxic potential, and that the NES values of the RNAs analyzed are added and compared to the added NES values of mRNAs determined for cells cultivated under the same cell culture conditions for the same duration in the absence of the agent.


SEQUENCE LISTING

<110> Medizinische Hochschule Hannover

<120> Analytical process for genotoxicity assessment

<130> M1066EP

<150> EP15202623.3
<151> 2015-12-23

<160> 78

<170> BiSSAP 1.3

<210> 1
<211> 60
<212> DNA
<213> Artificial Sequence

<220>
<223> probe for AFAP1L1

<400> 1
tagttatctt tgccttctga tgcaatgaac gcacgtaagc cattccatcc tttccttggc     60


<210> 2
<211> 60
<212> DNA
<213> Artificial Sequence

<220>
<223> probe for ALS2CL

<400> 2
ccctactttt ctgggtgatg gccacaatta cagtctactt atttattcag gatttttaat     60


<210> 3
<211> 60
<212> DNA
<213> Artificial Sequence

<220>
<223> probe for ARX

<400> 3
gtgtaattgt tatcactttt ccttgctatc tagtggagaa gtgtcacgct caaaataaaa     60


<210> 4
<211> 60
<212> DNA
<213> Artificial Sequence

<220>
<223> probe for BEX6

<400> 4
atgcttactg atgccatacc gacaatagcc aagatatgca gtcagcttgg aggttcaaca     60

<210> 5
<211> 60
<212> DNA
<213> Artificial Sequence

<220>
<223> probe for CCDC102A

<400> 5
tttcacctat cacacccctg tgaaggggga gcctgttgga tatgtatatt gtatatatgc     60


<210> 6
<211> 60
<212> DNA
<213> Artificial Sequence

<220>
<223> probe for DCTD

<400> 6
gcactgttaa atttatccga ggcataagta gttcctagag gagacagtct atcactcccc     60


<210> 7
<211> 60
<212> DNA
<213> Artificial Sequence

<220>
<223> probe for EHD3

<400> 7
gcttataact gcagtgtgtt tgtcattagg attcatgtta atacaacata tttaccctcg     60


<210> 8
<211> 60
<212> DNA
<213> Artificial Sequence

<220>
<223> probe for GM805

<400> 8
attttgatga ttcaagaaag cagaaggccc aaccttcagt aaaaccacag gtctccggga     60


<210> 9
<211> 60
<212> DNA
<213> Artificial Sequence

<220>
<223> probe for GUCY1A3

<400> 9
ggaactttat ctaattcaag tcaaaagaag tatagaagct ccatgtacgg ttcctataac     60


<210> 10
<211> 60
<212> DNA

<213> Artificial Sequence

<220>
<223> probe for MEX3A

<400> 10
gcaaagtaaa ggtgggaaat aaaacagacc catgaattaa tcaagtcaaa gtgatgttgc     60


<210> 11
<211> 60
<212> DNA
<213> Artificial Sequence

<220>
<223> probe for MYCT1

<400> 11
gtccttttgc ttcaaagaca ggagttcact gggggattca gaaagcttgc cgtttatgtt     60


<210> 12
<211> 60
<212> DNA
<213> Artificial Sequence

<220>
<223> probe for NAIP1

<400> 12
aatctatgag ttttatgaac tctctagggg gcttaacaac ccctcactgg gcagtgtcat     60


<210> 13
<211> 60
<212> DNA
<213> Artificial Sequence

<220>
<223> probe for NOLC1

<400> 13
ttcgaagtaa gcttttctga cagacatttt gcaacaactt gactgttgta tattgacaag     60


<210> 14
<211> 60
<212> DNA
<213> Artificial Sequence

<220>
<223> probe for PA2G4

<400> 14
gaatggatcc tcatttgtaa aaatttttag ccatttttgt tctgaacagt ccctcaatcc     60


<210> 15
<211> 60
<212> DNA
<213> Artificial Sequence

<220>

<223> probe for PDGFRB

<400> 15
ccacatttag acaccggaag tgctatttta tatgctgtta agttttccta tctgtacttt     60


<210> 16
<211> 60
<212> DNA
<213> Artificial Sequence

<220>
<223> probe for PHLDA2

<400> 16
accattccgt gttaatattt tttataccat attttcattc caaataaaca atgtcacttt     60


<210> 17
<211> 60
<212> DNA
<213> Artificial Sequence

<220>
<223> probe for SLA2

<400> 17
caaagataca cctccacctg tgactgtgcc aacatcatca ctaaattgga aaaagctgga     60


<210> 18
<211> 60
<212> DNA
<213> Artificial Sequence

<220>
<223> probe for SLC12A2

<400> 18
ctgctttgta cagaagttac tgcaataaag gaagtggagt catcacaagt ttaatactta     60


<210> 19
<211> 60
<212> DNA
<213> Artificial Sequence

<220>
<223> probe for SOX14

<400> 19
tggaccctgc gcaatttagt tccagtgcca tccagaagat gggtgaagtg ccccacacgt     60


<210> 20
<211> 60
<212> DNA
<213> Artificial Sequence

<220>
<223> probe for SOX4

<400> 20

aatcctgtcc gtcactgcct gtcaggttgt tcctatatac cttctgtaaa taactttttt     60

<210> 21
<211> 60
<212> DNA
<213> Artificial Sequence

<220>
<223> probe for SPNS2

<400> 21
gctgctgatt gtgaatctca aagtcttaag agagaagcca aatatatatt cctcttgtaa     60

<210> 22
<211> 60
<212> DNA
<213> Artificial Sequence

<220>
<223> probe for TBXA2R

<400> 22
tgagtgcttg gtggactaag gacggagcta cgacatgggg ctgactcctg tgaactgcaa     60

<210> 23
<211> 60
<212> DNA
<213> Artificial Sequence

<220>
<223> probe for TC1649157

<400> 23
tagagagagt gaaaccttgt tcttgctgag tcttcgagaa agaggaccaa gtcccaaagt     60

<210> 24
<211> 60
<212> DNA
<213> Artificial Sequence

<220>
<223> probe for TIE1

<400> 24
gtgaacatgt cgctgtttga gaacttcacc tatgcgggca tcgatgccac agctgaggag     60

<210> 25
<211> 60
<212> DNA
<213> Artificial Sequence

<220>
<223> probe for TMEM176A

<400> 25
ggtattttct cggagatgat gtctgtcaaa gagactcttc atatggatgg tccaccatgc     60

<210> 26
<211> 60
<212> DNA
<213> Artificial Sequence

<220>
<223> probe for TMEM176B

<400> 26
cctgaagaag tttctctcct ggcctcaggc cagaatccac tatgggcagc tgtctctagg     60


<210> 27
<211> 60
<212> DNA
<213> Artificial Sequence

<220>
<223> probe for TOX

<400> 27
tccaagtcct gttatggata aagctgccaa atgctcactg gcatcttagc tgcagaaaca     60


<210> 28
<211> 60
<212> DNA
<213> Artificial Sequence

<220>
<223> probe for ZFPM1

<400> 28
agtgagcctg tggggccgca ccaccaggac ccttgacact taataaagac attcggtgtg     60


<210> 29
<211> 60
<212> DNA
<213> Artificial Sequence

<220>
<223> probe for AJUBA

<400> 29
ggaagctgga gagaagaaaa ataggttgac ttggttctag ccgcttggct gaaacttgaa     60


<210> 30
<211> 60
<212> DNA
<213> Artificial Sequence

<220>
<223> probe for BHLHA15

<400> 30
tccctgagta ctagtaaaat tgccatctgt acctgaaaat gaagacttca cagccattgt     60


<210> 31
<211> 60
<212> DNA

<213> Artificial Sequence

<220>
<223> probe for CP

<400> 31
tctgatgtct ttgacctttt ccctggaaca taccaaaccc tagaaatgtt tccccaaaca     60


<210> 32
<211> 60
<212> DNA
<213> Artificial Sequence

<220>
<223> probe for ENSMUST00000103558

<400> 32
taacgacaca tacatgaagt ttagctggct tacagtgcct gaaagggcaa tggggaaaga     60


<210> 33
<211> 60
<212> DNA
<213> Artificial Sequence

<220>
<223> probe for GLRP1

<400> 33
ggggaaaaaa gcatcatttc cattgttctt tgctctgtgc cttgaatctt ttaatcaatt     60


<210> 34
<211> 60
<212> DNA
<213> Artificial Sequence

<220>
<223> probe for GRTP1

<400> 34
aagcagatca ccaaagggga ctttgtgaca gagtgtcacg cattcatgca gaaaatcttt     60


<210> 35
<211> 60
<212> DNA
<213> Artificial Sequence

<220>
<223> probe for LCK

<400> 35
aatcttatgt ctctgtgtgt tctgtcctgg tgcctagcac acaccaggag ctcaataaaa     60


<210> 36
<211> 60
<212> DNA
<213> Artificial Sequence

<220>

<223> probe for NAP002592-003

<400> 36
ttccccttcg aaaataggtg ttatagtcaa gataatgtac ttgggtcata agacagtgtg     60


<210> 37
<211> 60
<212> DNA
<213> Artificial Sequence

<220>
<223> probe for PIGA

<400> 37
ccagtgtgct aaaacatgct catgtccagt aataaagatg agaacgctgt tttaatagtc     60


<210> 38
<211> 60
<212> DNA
<213> Artificial Sequence

<220>
<223> probe for PRMT7

<400> 38
tggagacatc accatggagt ttaggcttgc agacaccttg agctgatctc ttattgagaa     60


<210> 39
<211> 60
<212> DNA
<213> Artificial Sequence

<220>
<223> probe for SLC35F2

<400> 39
cagccaccag agtgccgcct tcagaaaact ttatgtaaat tgtttttgta taagaataat     60


<210> 40
<211> 60
<212> DNA
<213> Artificial Sequence

<220>
<223> probe for TGM2

<400> 40
aaagcgtgga agcctgtgtg ggggtcatca tcccaagtta gcccactcct acctctctgt     60


<210> 41
<211> 60
<212> DNA
<213> Artificial Sequence

<220>
<223> probe for TIMP3

<400> 41

atagtgactt ttggggatag atctgtctgg gagataatgt gagtccagga ggcattactc     60

<210> 42
<211> 60
<212> DNA
<213> Artificial Sequence

<220>
<223> probe for ZFP184

<400> 42
gggatggaga gcccattggg ctgttctgat ccaagtatgt tattaaaaca acattgtggc     60

<210> 43
<211> 60
<212> DNA
<213> Artificial Sequence

<220>
<223> probe for AKAP13

<400> 43
ttagtcgcac cttcagctac atcaggaata aaatgtccag cagcaagaag agcaaagtaa     60

<210> 44
<211> 60
<212> DNA
<213> Artificial Sequence

<220>
<223> probe for ASB2

<400> 44
cctggacaca ctgccgcttc ccggcaggct aatcagatac ttgaaatatg agaatacaca     60

<210> 45
<211> 60
<212> DNA
<213> Artificial Sequence

<220>
<223> probe for ATP9A

<400> 45
ttcaacttct acttcctgct tctcgcctgc tcgcagttcg tcccagagat gaggcttggc     60

<210> 46
<211> 60
<212> DNA
<213> Artificial Sequence

<220>
<223> probe for CCR7

<400> 46
cagccactga tacctttcct catgttctgc ttttgattca tatatctttt atgaagaaac     60

<210> 47
<211> 60
<212> DNA
<213> Artificial Sequence

<220>
<223> probe for CPLX2

<400> 47
ggaggagaag gagaaggaga aggagaagga gaaggagaag gagaaggaga aggagaagga     60


<210> 48
<211> 60
<212> DNA
<213> Artificial Sequence

<220>
<223> probe for DUSP2

<400> 48
gtgggcatct cgctgtaatt ggtgctgaaa agttatttgt gttcaactga catttaacgc     60


<210> 49
<211> 60
<212> DNA
<213> Artificial Sequence

<220>
<223> probe for ELOVL6

<400> 49
cctaggccct aaacccatat tcagagggaa aattcactat caagcctcac agcgaaatca     60


<210> 50
<211> 60
<212> DNA
<213> Artificial Sequence

<220>
<223> probe for EPOR

<400> 50
gaacgggatt ggtgaagcca tacttaaagt cagagctgac cttggccctc tgagcaggaa     60


<210> 51
<211> 60
<212> DNA
<213> Artificial Sequence

<220>
<223> probe for FGF3

<400> 51
gctgtatgct tcggatcact acaacgcaga gtgtgagttt gtggaacgga tccatgagct     60


<210> 52
<211> 60
<212> DNA

<213> Artificial Sequence

<220>
<223> probe for IGF1R

<400> 52
gctaaagcta aggccacttg agtctattac tctgcttttt tctagtagtt aaagcaccac     60


<210> 53
<211> 60
<212> DNA
<213> Artificial Sequence

<220>
<223> probe for ITK

<400> 53
actgcaggta tgtcttaccc tctgtggggg ccacaagtca atcattgctt atggaaaaaa     60


<210> 54
<211> 60
<212> DNA
<213> Artificial Sequence

<220>
<223> probe for JAKMIP1

<400> 54
ctgtaccaga gcaatctatt tattgcctac cgcttgtttt gcacttaata aaataagttg     60


<210> 55
<211> 60
<212> DNA
<213> Artificial Sequence

<220>
<223> probe for KLRB1F

<400> 55
ggtttgtatt gattgacact cgccaactag ttgactaata atggactaat gctgtgttcg     60


<210> 56
<211> 60
<212> DNA
<213> Artificial Sequence

<220>
<223> probe for LMO2

<400> 56
cttcattttg aggtgaggcg ccccacagat tgtttctata caaatgtaaa tcttaaaaaa     60


<210> 57
<211> 60
<212> DNA
<213> Artificial Sequence

<220>

<223> probe for MEF2C

<400> 57
agaggaatag tatgtctcct ggtgtaacac atagacctcc aagtgcaggt aacacaggta     60


<210> 58
<211> 60
<212> DNA
<213> Artificial Sequence

<220>
<223> probe for MID1

<400> 58
aggttggatg tcaccaccac aagggaaaac aaacacatgg gtttttcccc aacacgatta     60


<210> 59
<211> 60
<212> DNA
<213> Artificial Sequence

<220>
<223> probe for MND1

<400> 59
ggcaagagac ggaagaacga gccatgcttg caaaagaact ttcttcattt cgagaccaaa     60


<210> 60
<211> 60
<212> DNA
<213> Artificial Sequence

<220>
<223> probe for MYB

<400> 60
tgacagtgta tctactgcct tgtagcaaaa taaagctatc ctcttatttt acatacttcc     60


<210> 61
<211> 60
<212> DNA
<213> Artificial Sequence

<220>
<223> probe for MYC

<400> 61
aacagttgaa acacaaactc gaacagcttc gaaactctgg tgcataaact gacctaactc     60


<210> 62
<211> 60
<212> DNA
<213> Artificial Sequence

<220>
<223> probe for PIM2

<400> 62

cacgctacag ggatagatgg acatctgttg acctggttat acaggttgtt aaaaatccag 60

<210> 63
<211> 60
<212> DNA
<213> Artificial Sequence

<220>
<223> probe for PRDM16

<400> 63
ctggatacca aatgtaatct ttccgatgct acaatgaatt tatacacgag attgatatgc 60

<210> 64
<211> 60
<212> DNA
<213> Artificial Sequence

<220>
<223> probe for RAMP1

<400> 64
agacgctatg gtgtgactgg ggaaagacca tacagagcta tggggagctc acttactgca 60

<210> 65
<211> 60
<212> DNA
<213> Artificial Sequence

<220>
<223> probe for RASGRP2

<400> 65
tctgttagac caagaaggga accgcaggca cagcagcctc atcgacatcg agagtgtgtg 60

<210> 66
<211> 60
<212> DNA
<213> Artificial Sequence

<220>
<223> probe for SEMA7A

<400> 66
tcccttatct cttctcaatg cactttaata atgtaacata ttactaataa acaagctatt 60

<210> 67
<211> 60
<212> DNA
<213> Artificial Sequence

<220>
<223> probe for SH3KBP1

<400> 67
gccaattaaa ctaagaccaa ggtcgattga agtggaaaat gacttcctgc ccgtcgaaaa 60

<210> 68
<211> 60
<212> DNA
<213> Artificial Sequence

<220>
<223> probe for SLC43A1

<400> 68
tgcacacata tatgttacgc acatgcatat tgcatccttt cctgcaggag aaggactgag     60


<210> 69
<211> 60
<212> DNA
<213> Artificial Sequence

<220>
<223> probe for SMAD3

<400> 69
tataaacgcc cttctaataa acttttcacc gtaaagctcc tgagacagga gcacagtctg     60


<210> 70
<211> 60
<212> DNA
<213> Artificial Sequence

<220>
<223> probe for ST3GAL6

<400> 70
aatggtgatc aacttgactt aaaattgacc ctatggatcc aaaagatgat gatgctaaac     60


<210> 71
<211> 60
<212> DNA
<213> Artificial Sequence

<220>
<223> probe for TNFSF13B

<400> 71
cgttgaatct gatccaaacc aggaaatata acagacagcc acaaccgaag tgtgccatgt     60


<210> 72
<211> 60
<212> DNA
<213> Artificial Sequence

<220>
<223> probe for VARS

<400> 72
tgctcttgaa ctagctctga gcatcactcg agctgtgcgc tccctgcgtg ctgactacaa     60


<210> 73
<211> 20
<212> DNA

<213> Artificial Sequence

<220>
<223> forward primer for WPRE

<400> 73
gaggagttgt ggcccgttgt                                                    20


<210> 74
<211> 20
<212> DNA
<213> Artificial Sequence

<220>
<223> reverse primer for WPRE

<400> 74
tgacaggtgg tggcaatgcc                                                    20


<210> 75
<211> 19
<212> DNA
<213> Artificial Sequence

<220>
<223> probe for WPRE

<400> 75
ctgtgtttgc tgacgcaac                                                     19


<210> 76
<211> 22
<212> DNA
<213> Artificial Sequence

<220>
<223> forward primer for PTBP2

<400> 76
tctccattcc ctatgttcat gc                                                 22


<210> 77
<211> 22
<212> DNA
<213> Artificial Sequence

<220>
<223> reverse primer for PTBP2

<400> 77
gttcccgcag aatggtgagg tg                                                 22


<210> 78
<211> 20
<212> DNA
<213> Artificial Sequence

<220>

<223> probe for PTBP2

<400> 78
atgttcctcg gaccaacttg                                                    20